# EUROPEAN PATENT SPECIFICATION

(11) **EP 2 450 055 B1**
(45) Date of publication and mention of the grant of the patent: **03.01.2018**
(21) Application number: 09846731.9
(22) Date of filing: 30.06.2009
(51) Int. Cl.: A61K 39/00, C07K 16/28

(54) **METHOD FOR SUPPRESSING RENAL TUMOR GROWTH BY BLOCKING FIBROBLAST GROWTH FACTOR RECEPTOR**
VERFAHREN ZUR UNTERDRÜCKUNG DES NIERENTUMORWACHSTUMS MITTELS BLOCKIERUNG DES FIBROBLASTENWACHSTUMSFAKTOR-REZEKTORS
PROCÉDÉ DE PRÉVENTION DE LA CROISSANCE DE TUMEURS DU REIN PAR BLOCAGE DU RÉCEPTEUR DU FACTEUR DE CROISSANCE DE FIBROBLASTES

(43) Date of publication of application: 09.05.2012
(73) Proprietor: Obshestvo S Ogranichennoy Otvetstvennostiu "OncoMax", Moscow 119180 (RU)
(72) Inventor: TSIMAFEYEU, Ilya Valer'evich, Moscow 125635 (RU)
(74) Representative: Sloboshanin, Sergej
(86) International application number: PCT/EA2009/000004
(87) International publication number: WO 2011/000384

(56) References cited:
- WO-A1-94/10202
- WO-A1-2010/122090
- US-A1- 2005 147 612
- SUN H D ET AL: "Monoclonal antibody antagonists of hypothalamic FGFR1 cause potent but reversible hypophagia and weight loss in rodents and monkeys", AMERICAN JOURNAL OF PHYSIOLOGY: ENDOCRINOLOGY AND METABOLISM, AMERICAN PHYSIOLOGICAL SOCIETY, BETHESDA, MD, US, vol. 292, no. 3, 1 March 2007 (2007-03-01) , pages E964-E976, XP008085179, ISSN: 0193-1849, DOI: 10.1152/AJPENDO.00089.2006
- SASAKI SHIGERU ET AL: "Anti-Tumor Efficacy of Anti-FGFR1 Monoclonal Antibody for Human Hepatoma Cells", TUMOR BIOLOGY, vol. 29, no. Suppl. 1, 2008, page 82, XP009167679, & 36TH MEETING OF THE INTERNATIONAL-SOCIETY-OF-ONCOLOGY-AND-BIOM ARKERS; TOKYO, JAPAN; OCTOBER 05 -09, 2008 ISSN: 1010-4283
- FORTIN DALE ET AL: "Distinct fibroblast growth factor (FGF)/FGF receptor signaling pairs initiate diverse cellular responses in the oligodendrocyte lineage.", THE JOURNAL OF NEUROSCIENCE : THE OFFICIAL JOURNAL OF THE SOCIETY FOR NEUROSCIENCE 10 AUG 2005, vol. 25, no. 32, 10 August 2005 (2005-08-10), pages 7470-7479, XP002693226, ISSN: 1529-2401
- TSIMAFEYEU I ET AL: "7116 Expression of fibroblast growth factor receptors 1 and 2 in renal cell carcinoma (RCC)", EUROPEAN JOURNAL OF CANCER. SUPPLEMENT, PERGAMON, OXFORD, GB, vol. 7, no. 2, 1 September 2009 (2009-09-01), pages 427-428, XP026690255, ISSN: 1359-6349, DOI: 10.1016/S1359-6349(09)71449-1 [retrieved on 2009-09-01]
- TSIMAFEYEU I ET AL: "Does dose-dependent targeting of fibroblast growth factor (FGF) receptor 1 (FGFR1) impact on growth of renal cell carcinoma?", EUROPEAN UROLOGY SUPPLEMENTS, vol. 11, no. 1, February 2012 (2012-02), page E305, XP009167666, & 27TH ANNUAL CONGRESS OF THE EUROPEAN-ASSOCIATION-OF-UROLOGY; PARIS, FRANCE; FEBRUARY 24 -28, 2012 ISSN: 1569-9056
- YIANGOU C ET AL.: 'Down-regulation of a novel form of fibroblast growth factor receptor 1 in human breast cancer' BRITISH JOURNAL OF CANCER vol. 76, no. 11, 1997, pages 1419 - 1427, XP009083338
- DATABASE MEDLINE SIFFROI-FERNANDEZ S ET AL.: 'Acidic fibroblast growth factor (FGF-I) and FGF receptor 1 signaling in human Y79 retinoblastoma', XP008130549 Database accession no. 15767480 & ARCH OPHTHALMOL. vol. 123, no. 3, March 2005, pages 368 - 376
- DATABASE MEDLINE CHEN YI W ET AL.: 'Basic fibroblast growth factor and fibroblast growth factor receptor-i in human meningiomas', XP008149948 Database accession no. 15934314 & J HUAZHONG UNIV SCI TECHNOLOG MED SCI. vol. 25, no. 1, 2005, pages 75 - 77

## Description

### Brief description of the invention

The invention generally relates to the field of medicine, in particular to suppressing the growth of tumors, based on blocking the pathway of "human fibroblast growth factor / human fibroblast growth factor receptor 1 (domains II and IIIc)". This pathological pathway leads to an excessive proliferation of tumor cells and to the formation of new vessels accompanied by the growth of primary tumors and metastases. This pathway also represents an independent mechanism of tumor resistance to pharmaceutical preparations acting on other pathological pathways. Blocking the pathway of "fibroblast growth factor / fibroblast growth factor receptor 1" using various substances that neutralize the receptor by binding only with domains II and IIIc thereof results in the inhibition or slow-down of tumor growth. This receptor can also be used as a target for targeted delivery of diagnostic agents as it is present in large amounts in the cells of numerous tumors. The invention is advantageous in that it makes it possible to develop new agents for diagnosing and treating diseases related to excessive proliferation and neovascularization.

Sun HD et al., Am J Physiology, vol. 292, no. 3 (March 2007), pages E964-E976 describes human antibodies specific for fibroblast growth factor receptor 1 (FGFR1) that block binding to fibroblast growth factor 1 or 2 and inhibit ligand-induced receptor phosphorylation. Human antibodies capable of blocking ligand-dependent activation of FGFR1 are also described in US 2005/147612 A1. Sasaki Shigeru et al., Tumor Biol, vol. 29, no. Suppl. 1 (2008) page 82 describes the use of an anti-FGFRl antibody for the treatment of human hepatocellular carcinoma.

The invention provides a monoclonal antibody for use in a method of treatment renal cancer, wherein the antibody binds to fibroblast growth factor receptor 1 (FGFR1) and antagonizes the interaction of fibroblast growth factor and FGFR1, and wherein the antibody is capable of simultaneously binding to domains II and IIIc of FGFR 1 or wherein the antibody is capable of binding to a complex of FGFR1 and heparin sulfate.

In a further embodiment, the antibody is characterized in that the antibody inhibits the mitogenic activity of FGFR1 or its complex with heparan sulfate by not less than 90%.

In a further embodiment, the antibody comprises an amino acid sequence of the heavy chain Fc domain of any one of: IgA, IgD, IgE, IgG1, IgG2, IgG3, IgG4 or IgM.

In a further embodiment, the antibody comprises a human Fc domain and/or a mouse Fv domain.

In a further embodiment, the antibody is a human antibody.

In a further embodiment, the antibody in addition comprises a non-immunoglobulin polymer.

In a further embodiment, the antibody in addition comprises a cytotoxic moiety or an amino acid sequence of a cytokine. The cytotoxic moiety may be a polypeptide toxin. The cytotoxic moiety may be capable of acting as an Fc-effector or capable of recruiting immunocompetent cells. On one embodiment, the cytotoxic moiety is a polypeptide capable of binding complement.

### Detailed description of the invention

It is known that the basis for the development of malignant neoplasms is excessive cell proliferation and formation in a tumor of blood vessels, which "feed" the tumor (angiogenesis).

New blood vessels are formed from already existing endothelium. The formation of new blood vessels is an important component of many diseases and disorders, including such as tumor growth and metastasis, rheumatoid arthritis, psoriasis, atherosclerosis, diabetic retinopathy, retrolental fibroplasia, neovascular glaucoma, hemangiomas, immune rejection of transplanted cornea and other tissues, and chronic inflammations.

In the case of tumor growth, angiogenesis is particularly important during the transition from hyperplasia to neoplasia, as well as to supply nutrition to the growing solid tumor (J. Folkman et al. Nature; 339, 58 (1989). Angiogenesis also allows tumors to keep in contact with the host circulatory system: this allows to determine the direction of tumor cell metastasis. Evidence of the role of angiogenesis in tumor cell metastasis was obtained, in particular, from studies showing the relationship between the quantity and density of micro-vessels in invasive breast cancer and the actual existence of distant metastases (N. Weidner et al. New Eng. J. Med., 324: 1 (1991).

According to numerous available data, proliferation of tumor cells as well as endothelial cells may be caused by various naturally occurring polypeptides. One of those polypeptides is the family of fibroblast growth factors (FGF). FGF was first detected in extracts of the pituitary gland in 1973 (H. Armelin. PNAS 70, 9 (1973).

FGF belong to a family of heparin-binding polypeptides modulating the function of various cells. FGF have a strong influence on the proliferation and differentiation of tumor and endothelial cells. Currently, 23 members of the FGF family are known (FGF 1-23). Each family member has its own specific functional features. FGF type 1 and 2 (acidic and basic) are the best studied FGF. In order to have an impact on cells, FGF must bind with a receptor on its surface. There are four types of FGF receptors (FGFR 1-4). FRFR1 binds not only to FGF 1 and 2, but also to most other members of this family. Accordingly, the role of this receptor in signal transduction into the cell is considered the most significant.

FGFR1 consists of extramembranous, intramembranous and intracellular parts. The extramembranous part of the receptor consists of three domains (D I-III), similar to immunoglobulin. FGF usually interact with D II and III; heparan sulfate, which is involved in formation of the FGF/FGFR1 complex, interacts with D III. Alternative mRNA splicing contributes to the formation of several different FRFR1 variants on the cell surface (D Johnson, L. Williams. J Adv. Cancer Res., 60, 1 (1993); McKeehan et al. J Prog Nucleic Acid Res. Mol. Biol., 59, 135 (1998). The intracellular part of the receptor is represented by tyrosine kinases, the autophosphorylation of which results in the signal transduction further to the cell nucleus and cell division.

### Study of Renal Cancer by Bureau

When studying the effects of low molecular weight heparin (LMWH) in patients with metastatic renal cell carcinoma (RCC) in a randomized trial, we have proved that heparin does have impact on patient survival and on the response rate to immunotherapy. We hypothesized that LMWH can bind FGF, interact with its receptor (FGFR1), other heparan/heparin-binding factors (I. Tsimafeyeu et al. Rossiyskiy Oncologicheskiy Zhournal (Russian Journal of Oncology), 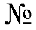 5 (2008); I. Tsimafeyeu et al. J. Clin. Oncology 25, 18S (2007)). In another study, we showed that in 40% of cases patients with metastatic RCC have hemostatic system disorders, which can also be caused by increased formation of FGF and expression of FGFR1 (I. Tsimafeyeu et al. J. Experimental & Clinical Cancer Research, 28 , 30 (2009)).

In our further studies of the KCRB-L01 and KCRB-L02 we investigated the role of the FGF/FGFR1 complex in the development of RCC.

KCRB-L01: Study of expression of FGFR in patients with RCC. Immunohistochemical studies were performed on sections from paraffin blocks of tumors of 140 patients with RCC. The results were compared with the expression of FGFR in 40 healthy donors who had previously had renal biopsy performed for various reasons without the subsequent discovery of diseases of the organ. We found expression of FGFR1 in 98% of cases on the cells in the primary renal tumor and in 82.5% of cases in the cells of metastatic RCC. In all cases, the intensity of staining in immunohistochemical analysis was high (3 +), which indicates strong expression of the receptor. In 68% of cases, nuclear staining was obtained. FGFR1 expression in cells of healthy renal tissue was found in 1 case (2.5%) due to the staining of blood vessels. Thus, this study confirmed the hypothesis about the existance and high expression of FGFR1 on the cells of the primary RCC tumor as well as in RCC metastases (I. Tsimafeyeu et al. ESMO-ECCO 09 (2009): Table 1.

In the study of KCRB-L02, we determined the concentration of FGF 1 and FGF 2, i.e., major factors having mitogenic activity in binding to FGFR1, in the blood plasma of 38 patients with metastatic RCC prior to the targeted therapy, during disease progression in the targeted therapy, as well as in blood plasma of 38 healthy volunteers (by the ELISA method). We found that in the blood of healthy individuals, levels of both FGFs were significantly lower as compared to patients with metastatic RCC (Table 2). The greatest differences were demonstrated for FGF 2 (p <0.001).

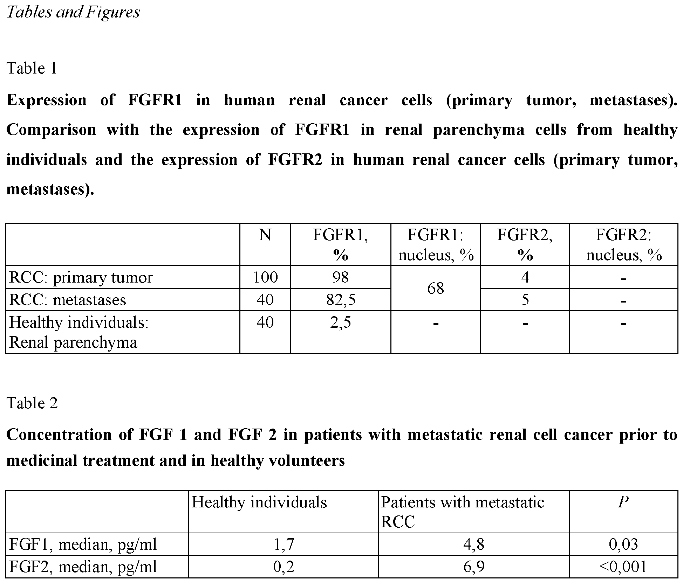

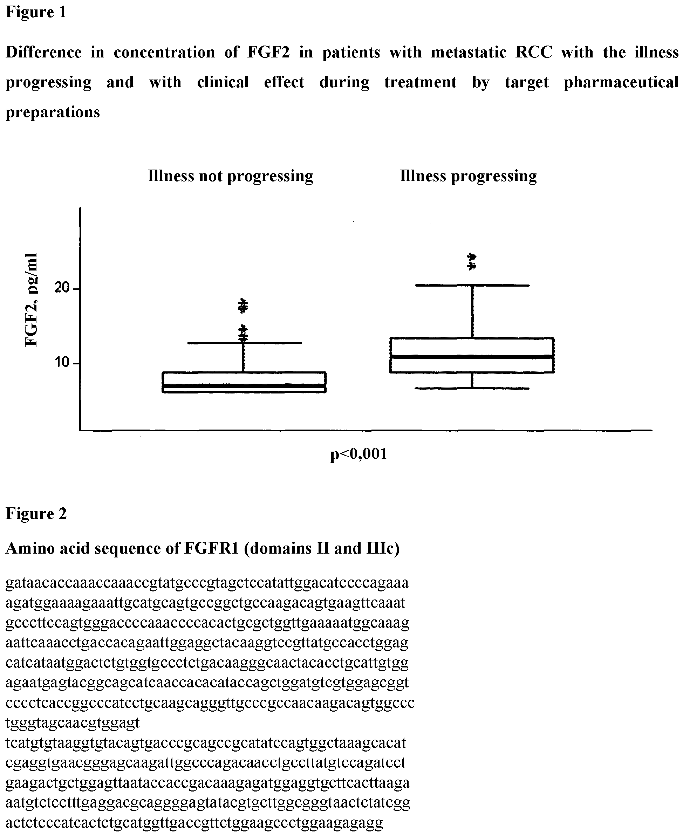

When the disease progressed during the targeted therapy (sunitinib, sorafenib) we observed a significant increase in FGF 2 - by more than 50% (p <0.001), and in FGF 1 - by more than 30% (p <0.05) as compared to the baseline FGF level. When the targeted therapy was effective, no significant change in the level of both FGFs was observed (p = 0.3). The median concentration of FGF 2 in the plasma of patients with and without disease progression differed significantly (p<0.001, Figure 1).

In addition, in this study we analyzed the level of the target for sunitinib / sorafenib, i.e., vascular endothelial growth factor (VEGF).

There were no statistical differences between the concentration of VEGF in the plasma of patients with RCC in case of disease progression and the original baseline (p = 0.2), and no correlation with both FGFs (p> 0.1).

Thus, the results of the KCRB-L01 and KCRB-L02 studies suggest that the pathological pathway of FGF/FGFR1 is not only independent of the development of RCC, but may determine the resistance to the existing targeted therapy of tumors.

Other authors have also demonstrated the role of FGF/FGFR1 in the development of such tumors as non small cell lung cancer, breast cancer, gastric cancer and esophageal cancer, prostate cancer, bladder cancer, head and neck tumors, melanoma (C. Behrens et al. J Clinical cancer research 14, 19 (2008); M. Koziczak et al. J Oncogene, 23,20 (2004); K. Freier J Oral Oncology 43, 1 (2007); E. Shin et al. J Cancer Res Clin Oncol. 126, 9 (2000); K. Sugiuraet al. J Oncology reports 17, 3 (2007); E. Devilard et al. J BMC Cancer 6, 272 (2006); G. Lefevre et al. J Investigative Ophthalmology and Visual Science 50 (2009)).

Based on the foregoing, we hypothesized that blocking the FGF/FGFR1 path may disturb tumor cell proliferation and inhibit angiogenesis. Antagonists of FGFR1, including human monoclonal antibodies, can be used to suppress tumor growth and metastasis. In addition, conjugates of a monoclonal anti-FGFRl antibody (or its fragments) and a contrast agent can be used in the diagnosis of malignant neoplasms as well as other neoplasms whose cells express FGFR1 in large quantities.

An object of the present invention is to provide a method of suppressing tumor growth by blocking (neutralization) of domains II and IIIc of FGFR1, as well as a method of diagnosing tumors whose cells express FGFR1.

To test the hypothesis and to achieve the objectives, we conducted studies KCRB-L03, KCRB-L04 and KCRB-L05.

In all these, as FGFR1 antagonists we used synthesized highly specific neutralizing monoclonal antibodies 1) against the domains FGFR1 II and IIIc (10-1); and 2) against FGFR1 and heparan sulfate (IO-2) to block FGFR1 (hereafter FGFR1 refers to a receptor corresponding to registration numbers in international databases Uniprot - PI 1362 and Entrez - 2260, namely, its domains II and IIIc (amino acid sequence shown in Figure 2)).

The term "monoclonal antibody" is used herein and below to denote an antibody obtained from a population of sufficiently homogeneous antibodies, i.e., individual antibodies within the population are identical in their specificity and affinity, except for possible naturally occurring mutations that may be present in minor amounts.

Attention should be paid to the fact that as a result of similar, naturally occurring mutations the content of the monoclonal antibodies of the present invention, which pertains to antibodies that specifically bind the FGFR1 or the FGFR1/heparan-sulfate complex or the FGF/FGFR1 complexes or inhibit the binding of FGF with FGFR1, may not be entirely homogeneous.

Thus, the term "monoclonal" indicates the type of antibody which originates from a sufficiently homogeneous population of antibodies. This, however, does not mean that the antibodies must be produced by any particular method. For example, monoclonal antibodies described herein can be produced by the hybridoma method (G. Köhler, C. Milstein. J Nature 256, 495 (1975) or by employing methods involving recombinant DNA (S. Cabilly et al. US Patent N 4816567)).

To produce monoclonal antibodies by a hybridoma, mouse or another appropriate host animal is immunized with an antigen by subcutaneous, intraperitoneal, or intramuscular injection in order to identify lymphocytes that produce or are capable of producing antibodies which specifically bind to the protein(s) used for immunization. Alternatively, lymphocytes may be immunized in vitro. Then the lymphocytes are fused with myeloma cells using an appropriate agent, such as polyethylene glycol, to create a hybridoma cell (J. Goding. Monoclonal Antibodies: Principles and Practice, pp. 59-103 (Academic Press, 1986)).

In this invention, such an antigen is FGFR1 (domains II and IIIc) or an FGFRl/heparan-sulfate complex. The antigen may be a fragment or portion of FGFR1, having one or more amino acid residues that are involved in the binding of FGF.

The hybridoma cells thus prepared were seeded and grown in a suitable culture medium that preferably should contain one or more substances that inhibit the growth or survival of the unfused, parental myeloma cells. For example, if the parental myeloma cells do not contain the enzyme hypoxanthine guanine phosphoribosyl transferase (HGPRT or GPRT), culture medium for hybridomas will typically include hypoxanthine, aminopterin and thymidine (HAT medium), i.e., those substances that prevent the growth of cells that do not have HGPRT.

It is preferable to choose such myeloma cells that fuse efficiently, support a stable high level expression of antibodies in selected cells producing antibodies, and which are sensitive to the media, such as, for example, the HAT medium.

Among such cells the preferred cell lines are mouse myeloma cell lines, such as cell lines derived from murine tumors MPC-21 and MPC-11, which can be obtained from the Center for the distribution of cells of Salk Institute, San Diego (California, USA), SP-2 cells, which can be obtained from American Type Culture Collection in Rockville (Maryland, USA) and cells P3X63Ag8U.1, described by Yelton et al. (J Curr. Top. Microbiol . Immunol. 81, 1 (1978)). Cell lines from human myeloma and mouse-human-heteromieloma, which are capable of producing human monoclonal antibodies have also been described (D. Kozbor et al. J Immunol. 133, 3001 (1984); B. Brodeur, P. Tsang Monoclonal Antibody Production Techniques and Applications, pp. 51-63 (Marcel Dekker Inc., New York, 1987)).

The cultural medium in which the hybridoma cells are grown is analyzed for the production of monoclonal antibodies directed against the relevant antigen. The specificity of binding the monoclonal antibodies produced by hybridoma cells should preferably be high. The present invention includes those monoclonal antibodies (10-1/IO-2), which showed high specificity (at least 5x10⁹) of binding to these antigens, determined by the standard method of «BIOCORE». In other words, the antibodies bind at least one of these antigens according to the analysis for binding and can inhibit biological activity of the FGFR1. High specificity and strong inhibition of the pathway are ensured by simultaneous binding to domains II and IIIc of this receptor.

After identifying those hybridoma cells that produce antagonistic antibodies of desired specificity, affinity and activity, the clones may be subcloned by limited dilution and grown by standard methods (J. Goding. Monoclonal Antibodies: Principles and Practice, pp. 59-103 (Academic Press, 1986)). Suitable culture media include, for example, the Needle medium, a modified Dulbecco (SIMD), or RPMI-1640 medium. In addition, the hybridoma cells can be grown in vivo in animals as ascites tumors.

Monoclonal antibodies produced by subclones are separated from the culture medium, ascites fluid or plasma by using conventional methods of immunoglobulin purification, such as, for example, protein A-Sepharose, hydroxylapatite chromatography, electrophoresis in gels, dialysis, or affinity chromatography.

DNA encoding monoclonal antibodies described in the present invention can readily be isolated and sequenced by conventional methods (e.g., using oligonucleotide probes capable of binding specifically to genes encoding the heavy and light chain of murine antibodies). Hybridoma cells were used as a DNA source. After being isolated, the DNA may be placed in expression vectors, which are then transfected into host cells such as simian COS cell line, Chinese hamster ovary cells (CHO) or myeloma cells that otherwise do not produce immunoglobulins, in order to achieve the synthesis of monoclonal antibodies in recombinant host cells.

DNA can be modified in order to change the nature of the immunoglobulin produced by the DNA expression. For example, humanized forms of mouse antibodies can be obtained by replacing the complementarity determining region (CDR) of the variable domain of a mouse antibody by the corresponding region of a human antibody. In some variants, individual amino acids from the fundamental region (FR) of a mouse antibody are also replaced by the corresponding amino acid residues of a human antibody (P. Carter et al. Proc. Nat. Acad. Sci. 89, 4285 (1992); P. Carter et al. J Biotechnology 10, 163 (1992)). Chimeric forms of murine antibodies can also be obtained by the substitution of homologous murine DNA sequences by the sequence encoding a specific area of human immunoglobulin constant chains (heavy and light) (S. Cabilly et al. US Patent N 4816567; S . Morrison et al. Proc. Nat. Acad. Sci. 81, 6851 (1984)).

The antibodies used to solve the objectives of the present invention include mouse antibodies (IgG). However, other forms of antibodies can also be obtained such as "humanized" as well as fully human antibodies. This only reflects the percentage of human protein and does not affect the specificity of binding to the antigen, i.e., proof of the method of the present invention.

In addition, for blocking FGFR1 and its domains various classes and types of antibodies (e.g., IgA, IgD, 1gE, IgG and IgM), immunoglobulin subclasses as well as antibody fragments (e.g., Fab, F(ab')2, and Fv) can easily be obtained, as long as they show the ability to bind to FGFR1 and as long as they exhibit antagonism to biological activity of the FGF/FGFR1 pathway according to the present invention.

In a preferred embodiemnt of the invention, monoclonal antibodies will have affinity for the immunizing antigen of at least 10⁹ liters/mol (P. Munson, D. Rodbard. J Anal. Biochem. 107, 220 (1980)).

In addition, monoclonal antibodies will usually inhibit the mitogenic or angiogenic activity of FGFR1 by at least 90%, as determined, for example, in the analysis of cell survival or cell proliferation in vitro, similar to that described in our studies KCRB-L03 (Example 1) and KCRB-L04 (Example 2).

For therapeutic and diagnostic application, it is desirable that monoclonal antibodies of the invention do not react with all components and molecular forms of FGFR1. For example, it is desirable to obtain a monoclonal antibody that specifically binds only to the II and IIIc domains of FGFR1, and not to domain I or other domains and isoforms of the receptor. For this, the extracellular part of FGFR1 including domains II and IIIc was used for immunization. Necessary molecular forms of antibodies can easily be determined by comparing ELISA or by comparing immunoprecipitation of different polypeptides FGFR1. This makes possible the immunization by different FGFR1 isoforms.

FGFR1 can also be blocked by other known methods, in particular, by chemically synthesized inhibitors.

### Therapeutic use of the process of blocking the FGF/FGFR1pathway

For therapeutic use according to the invention, FGF/FGFR1 pathway antagonists, *i.e.* the monoclonal antibodies of the invention, are delivered to a mammal, preferably a human, in a pharmaceutically acceptable form, including intravenous administration, as well as by following ways: intramuscular, intraperitoneally, intracerebrospinally, subcutaneously, intraarticular, intrasinovially, intrathecally, orally, locally or by inhalation.

Antagonists of the invention can also be administered into the tumor, near the tumor, into the lesion or near the lesion, to ensure local action, along with systemic therapeutic effects. Such forms of administration require pharmaceutically acceptable carriers, which by their nature do not have any toxic or therapeutic effects. Examples of such carriers are ion-exchange material, alum, aluminum stearate, lecithin, plasma proteins (such as human plasma protein), buffer substances such as phosphates, glycine, sorbic acid, potassium sorbate, partial glyceride mixtures of saturated vegetable fatty acids, water, salts or electrolytes, such as protamine sulfate, sodium hydrophosphate, potassium hydrophosphate, sodium chloride, zinc salts, colloidal silica, magnesium trisilicate, polyvinyl pyrrolidone, cellulose base material and polyethylene glycol. Carriers for local or gel-based forms of antagonist include polysaccharides such as sodium carboxymethylcelluloses or methylcelluloses, polyvinylpyrrolidone, polyacrylates, polymers of polyoxiethylene polyoxipropylene block, polyethylene glycol, and alcohols.

Conventional dosage forms obtained from stocks are used for administration. Such forms include, for example, microcapsules, nanocapsules, liposomes, plasters, inhalation preparations, sprays, sublingual tablets and medicines with constant release of substance. In these preparations the antagonist will usually be present in a concentration of about 0.1 mg/ml to 100mg/ml.

Suitable examples of drugs with permanent release agents include semipermeable matrices of solid hydrophobic polymers containing the antagonist; such matrices have a definite shape, for example it may be a film or microcapsule. Examples of matrices with constant release include polyethers, hydrogels [e.g., poly (2-hydroxyethyl methacrylate)] as described by Langer et al (J. Biomed. Mater. Res. 15, 167 (1981)) and Langer (Chem. Tech. 12 (1982)), or poly(vinyl alcohol), polylactides (U.S. Patent 3773919), copolymers of L-glutamic acid and gamma ethyl-L-glutamate, described by Sidman et al (Biopolymers 22, 547 (1983)), nondegradable ethylene vinyl acetate (Langer et al., see above), degradable copolymers of lactic and glycolic acids, such as the Lupron Depot ™ (injectable microspheres composed of polymers of lactic and glycolic acids and acetate leuprolide), and poly-D-(-)-3-hydroxibutyl acid. While polymers such as ethylene vinyl acetate and the copolymer of lactic and glycolic acids are capable of continuous release of molecules for over 100 days, certain hydrogels release proteins for shorter periods of time. When the encapsulated polypeptide antagonists remain in the metabolism for a long time, they can denature or aggregate as a result of exposure to moisture at 37°C, which leads to the loss of biological activity and possible changes in immunogenicity. Appropriate strategies can be designed for stabilization of the antagonists, depending on the current mechanism. For example, in case of aggregation by formation of intermolecular S-S bonds through thiodisulfide exchange stabilization can be achieved by modifying sulfhydryle residues, by lyophilization to remove acidic solutions, by humidity control, by using appropriate additives, and developing specific polymer matrix compositions.

Antagonistic compounds with a constant release of anti-FGFRl agent also include antagonistic antibodies that are enclosed in liposomes. Liposomes containing the antagonist may be prepared by methods known in the field, for example, described by Epstein et al. (Proc. Nat. Acad. Sci. 82, 3688 (1985)), Huang et al. (Rgoss. Nat. Acad. Sci. 77, 4030 (1980)) U.S. Patent 4,485,045 and U.S. Patent 4,544,545.

Liposomes tend to have small size (of about 200-800 angstroms) and belong to a single-layer type in which the lipid content is higher than 30 mol.% cholesterol; the ratio can be varied in order to select optimal conditions for therapy. Liposomes with long circulation are covered by U.S. Patent 5,013,556.

According to another aspect of the invention, an FGF/FGFR1 pathway antagonist is incorporated into products which have a define shape. Such products can be used to modulate the growth of endothelial cells and angiogenesis. In addition, such products can be used to modulate tumor invasion and metastasis.

It is also possible to conjugate an FGF/FGFR1 pathway antagonist and another therapeutic means. For prevention or treatment of a disease the required dose of antagonist will depend on the type of disease, on its severity and course, on whether the antibodies are introduced with preventive or therapeutic purposes, on the previous therapy, on the patient's anamnesis and his response to the antagonist, and on instructions from the attending physician. The antagonist may be administered to the patient in various ways, once or as a series of prescriptions.

FGF/FGFR1 antagonists can be used to treat various neoplastic and non-neoplastic diseases and disorders. Neoplasms and similar conditions that are amenable to such treatment, include renal cell cancer, lung cancer, stomach cancer, esophageal cancer, colorectal cancer, liver cancer, ovarian cancer, cervical cancer, endometrial cancer, endometrial hyperplasia, endometriosis, fibrosarcomas, horiosarkomas, tumors of the head and neck, hepatoblasts, Kaposi's sarcoma, melanoma, skin cancer, hemangioma, cavernous hemangioma, hemangioblastomas, pancreatic cancer, retinoblastoma, astrocytoma, glioblastoma, Schwan, oligodendroglioma, medulloblastomu, neuroblastoma, rabdomiosarkomu, osteogenic sarcoma, LMS, bladder cancer and other urothelial tumors, Wilms' tumor, prostate cancer, abnormal proliferation of blood vessels associated with phacomatoses. According to the invention, FGF/FGFR1 antagonists, *i.e*., monoclonal antibodies to domains II and IIIc of FGFR1 or a complex of FGFR1 and heparin sulfate, are for use in a method of treatment of renal cancer.

FGF/FGFR1 antagonists can also be used for to treat non-neoplastic diseases including such as rheumatoid arthritis, psoriasis, atherosclerosis, diabetic retinopathy and other retinopathies, fibroplasia, neovascular glaucoma, thyroid hyperplasias (including Grave's disease), transplantation of the cornea and other tissues, chronic inflammations, lung inflammation, nephrotic syndrome, ascites, preeclampsia, pericardial effusion (for example, associated with pericarditis) and pleural effusion. Depending on the type of the disease and its severity, the initial dose administered to the patient will range from 1 mg/kg to 15 mg/kg and can be administered by a single or a series of individual injections or by a continuous infusion. The usual daily dose may vary from about 1 mg/kg to 100 mg/kg or more, depending on the factors mentioned above. Depending on conditions, the treatment is repeated after a few or more days until the desired suppression of symptoms is achieved. Other regiments of dosages can be used. The success of treatment can easily be determined by conventional methods and analyses, such as X-ray imaging of tumors.

According to another aspect of the invention, the efficiency of an FGF/FGFR1 pathway antagonist in preventing or treating disease may be improved by administering the antagonist serially or in combination with another substance effective for this purpose, such as tumor necrosis factor, interferons, interleukins, antibodies and inhibitors able to neutralize or inhibit the angiogenic activity of endothelial growth factor of blood vessels and its receptors and/or hepatocyte growth factor and/or epidermal growth factor and its receptors and/or placental growth factor and/or mTOR and/or other intracellular kinases, or one or more of the conventional therapeutic agents such as, for instance, alkylating agents, folic acid antagonists, antimetabolites of nucleic acid metabolism, antibiotics, pyrimidine analogs, 5-flyuorouracil, purine nucleosides, amines, amino acids, triazole nucleosides, or corticosteroids. Such substances may be present in the administered composition, or can be administered separately. In addition, an FGF/FGFR1 pathway antagonist can be administered serially or in combination with radiological treatments, which may include both irradiation and introduction of radioactive substances.

In accordance with one aspect of the invention, tumor vascularization is under attack in a combination therapy. One or more of FGF/FGFR1 antagonists are administered to the patient with a tumor in a therapeutically effective dose, defined, for example, by observing tumor necrosis or its metastatic foci, if any. This therapy continues as long as there is no further improvement observed or a clinical examination shows that the tumor or its metastases have disappeared. With the progression of the disease one or more of the aforementioned substances is introduced, or hyperthermia, or radiation therapy are applied.

Because the effectiveness of additional agents will vary, it is desirable to compare their effect on the tumor by standard screening matrix. An FGF/FGFR1 antagonist as well as the additional agent is re-administered until the desired clinical effect is reached. Alternatively, FGF/FGFR1 antagonist(s) are introduced together and, if desired, together with additional substances.

### Diagnostic use

Antibodies to FGFR1 and to its domains II and IIIc can be used in diagnosis. Antibodies will generally be marked with a residue which is easy to detect. This can be any residue which directly or indirectly may produce a detectable signal. For example, this may be radioisotopes such as 3H, 14C, 32P, 35S, 1251; fluorescent or chemiluminescent compounds such as fluorescein isothiocyanate, rhodamine, or luciferin; tags marked with radioisotopes, such as, for example, 1251, 32P, 14C or 3H, or enzymes such as alkaline phosphatase, beta galactosidase or horseradish peroxidase.

Any method known in the field can be used herein for the conjugation of the various antibodies to detectable residues, including the methods described by Hunter et al. (J Nature 144, 945 (1962)), David et al. (J Biochemistry 13,1014 (1974)), Payne et al. (J Immunol. Meth. 40, 219 (1981)) and Nigren (J. Histochem. and Cytochem. 30, 407 (1982)).

Antibodies of the present disclosure or FGFR1 can be used in the diagnosis of tumors in humans and mammals. For this, an antibody or FGFR1 marked with a detectable residue is introduced into a patient, preferably into the circulatory system, and the presence and location of the marked antibody or receptor in the patient's body is analyzed. Such visualization can be used, for example, in determining the stage of a disease or the treatment of neoplasms. The antibody or FGFR1 are marked with any residue found in mammals by known methods, such as nuclear magnetic resonance, radiology, etc.

Examples of means for suppressing tumor growth by inhibiting (neutralizing) FGFR1, as well as diagnosing tumor whose cells express FGFR1 are set forth below. The examples are offered only as an illustration of the invention and should not limit the invention.

### Example 1 (Results of KCRB-L03 study): analysis of survival or cell proliferation in vitro, impairment of FGFR1 function by adding monoclonal antibodies blocking FGFR1 domains II and IIIc.

To select a model cell line different cell lines were studied for hyperexpression of FGFR1 :
1) human renal cancer Caki-1
2) human breast cancer MCF7
3) human prostate cancer Dul45
4) human non small cell lung cancer A549

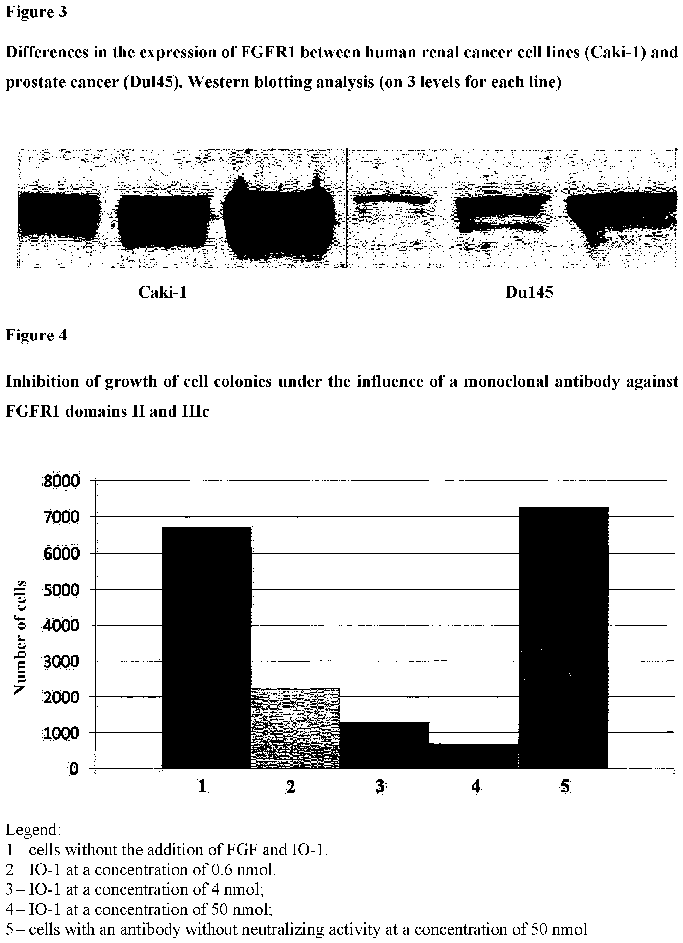

Hyperexpression of FGFR1 on the cells was determined by a standard Western blotting analysis. The highest overall level of expression of FGFR1 on cells was 40%. The highest level of expression of FGFR1 was detected in the human renal cancer cell line Caki-1, the lowest level - in the cells of the human prostate cancer Du145 (10 fold difference): Figure 3.

Based on these data, human renal cancer cell line Caki-1 was selected for further studies.

Cells were seeded with a density of 10⁴ cells/ml in 6-well plates. In each well there were added neutralizing monoclonal antibodies IO-1 and IO-2 in equal volume but in different concentrations. For control, a non-related monoclonal antibody without neutralizing capacity (Abcam) was added to a part of the culture. After incubation, 10 ng/ml FGF 2 was added to each well. As an additional control, some cells were grown in the absence of both antibodies and FGF 2. After the growth of the culture for 3 weeks, the cells in each well were counted by a computer program on the Hewlett Packard Scanjet analyzer (U.S.).

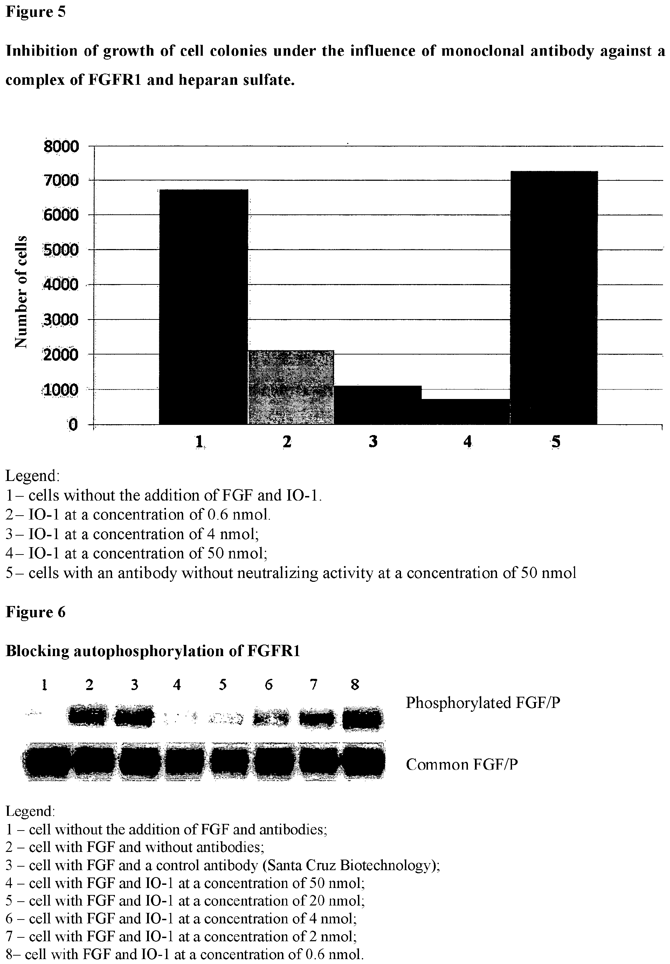

As shown in Figures 4 and 5, both monoclonal antibodies (IO-1 and 10-2) completely inhibited the ability of the added FGF 2 to support the growth and survival of renal cancer cell line (more than by 90%). No significant difference in the activity of IO-1 and 10-2 was revealed. The monoclonal antibody to FGFR1 without neutralizing capacity (Abcam) did not cause any changes in cell survival. The experiment revealed that the suppression of mitogenic activity depends on the dose of the agent that blocks the FGF/FGFR1 pathway (the higher the dose, the higher the mitogenic activity). The general conclusion from this example is that simultaneous blocking of FGFR1 domains II and IIIc results in a strong inhibition of tumor cell growth.

In this example we also show that blocking the fibroblast growth factor receptor leads to the disruption of receptor autophosphorylation, which reflects its functional importance.

For this, antibody IO-1 was added to the cells described above; 10 ng/ml FGF 2 were added 1.5 hours later. The cells were cultured for 5 minutes at a temperature of 37°C. Then the cells were washed and lysed in a special buffer for lysis (50 mM HEPES (pH 7.4), 150 mM NaCl, 10% glycerol, 1% Triton X-100, 1.5 mM MgCl2, protease inhibitors and 2 mmol of sodium vanadate). The lysate was incubated on ice for 30 minutes, and then centrifuged (13,000 rpm for 10 min at 4°C). The concentration of protein in the lysate was measured by Coomassie Plus analysis (Pierce). Afterwards immunoprecipitation / Western blotting was conducted. These techniques were performed by standard protocol (Santa Cruz Biotechnology, USA) using 1 mg monoclonal antibody to bind to FGFR1 (control antibody; Santa Cruz Biotechnology), and anti-phosphotyrosine (4G10) antibodies. The resulting sample was used for electrophoresis and subsequent identification of co-precipitated proteins in Western blotting. Cells without the addition of FGF 2 and antibodies were used as control.

The results are shown in Figure 6. It is shown that the monoclonal antibody IO-1 disturbs the process of FGFR1 phosphorylation, whereas the control anti-FGFRl antibody (Santa Cruz Biotechnology, USA) did not inhibit the phosphorylation of receptor. The blocking agent (in this case IO-1) concentration affects the phosphorylation of FGFR1: the higher the concentration, the greater the effect.

Thus, Example 1 (study KCRB-L03) shows that the cells of human renal cell cancer proliferate in the presence of FGF 2, but cease to proliferate and lose their mitogenic activity when the target receptor FGFR1 (only domains II and IIIc) is blocked and its function is inhibited. It is also demonstrated in Example 1 that in the absence of FGF 2 the cells do not proliferate either, evidencing its mitogenic role (if FGF 2 is blocked, the cells do not proliferate any longer).

### Example 2 (Results of KCRB-L04 study)): analysis of survival or proliferation of endothelial cells in vitro, impaired function of FGFR1 on endothelial cells after adding a monoclonal antibody that blocks FGFR1

To analyze the survival of endothelial cells in the FGF medium and after blocking FGFR1 an experiment similar to the blocking of vascular endothelial cells growth factor, as described in U.S. patent 20090022716 (Rockwell; Patricia et al. US Patent 20090022716) was conducted.

Bovine capillary endothelium of the adrenal cortex (CEAC) (N. Ferrara et al. Proc. Nat. Acad. Sci. 84: 5773 (1987) was used as model endothelial cells.

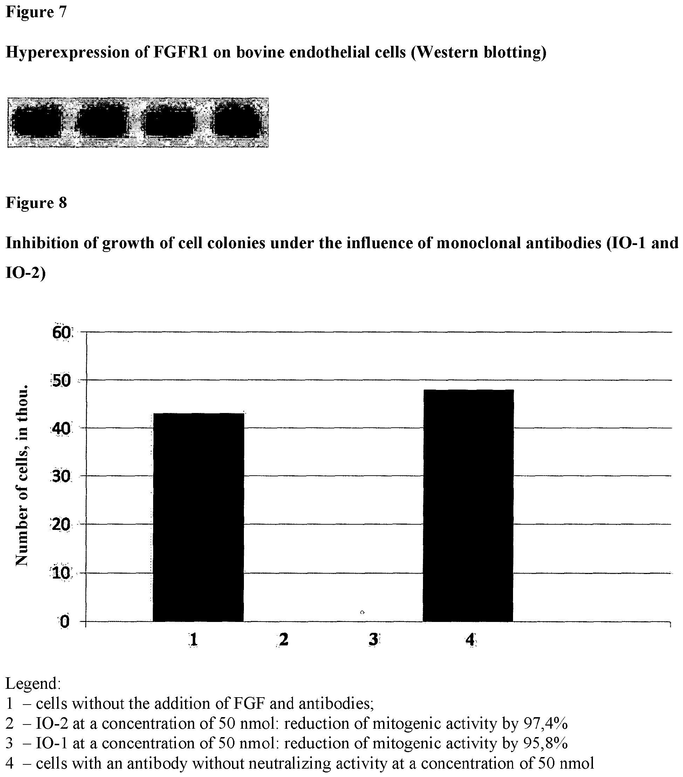

We first identified high expression of FGFR1 in the standard CEAC Western blotting analysis (figure 7).

Then CEAC were planted with a density of 5x10⁴ cells / ml in 12-well plates. 10 ng/ml FGF 2 was added into each well in the presence or absence of various concentrations of monoclonal antibodies to FGFR1 as well as an unrelated monoclonal antibody without FGFR1 neutralizing activity (Abcam). After 5 days of culture growth the cells in each well were counted by a computer program for the Hewlett Packard Scanjet analyzer (U.S.). As an additional control, CEAC were grown in the absence of FGF 2.

As shown in Figure 8, both monoclonal antibodies to FGFR1 inhibited the ability of added FGF 2 to support the growth and survival of bovine CEAC.

The monoclonal antibody without neutralizing activity (Abeam) had no effect on cells.

Thus, Example 2 demonstrates that when the FGF/FGFR1 pathway is blocked endothelial cells cease to proliferate and lose their mitogenic activity. This may lead to inhibition of tumor angiogenesis.

### Example 3 (Results of KCRB-L05 study): Inhibition of tumor growth in vivo by blocking the FGF/FGFR1 pathway

2x10⁶ cells of human renal cancer cell line Caki-1 in 100 ml of physiological solution with phosphate buffer (FGFB) were subcutaneously injected into female mice (Beige / nude) aged 5-6 weeks (purchased from Harlan Sprague Dawley, Inc. (Indianapolis, USA)). Once tumor growth was established, mice were divided into 3 groups.

The first (treatment) group of mice was intraperitoneally injected with anti-FGFRl monoclonal antibody IO-1 at a dose of 100 mg/kg 2 times a week. The second (treatment) group of mice was intraperitoneally injected with a monoclonal anti-FGFRl/heparan-sulfate antibody IO-2 at a dose of 100 mg / kg 2 times a week Third (control) group of mice was injected with saline. Each group included 15 mice.

The size of the tumor was measured every 5 days, and at the end of the study tumors were extracted and weighed.

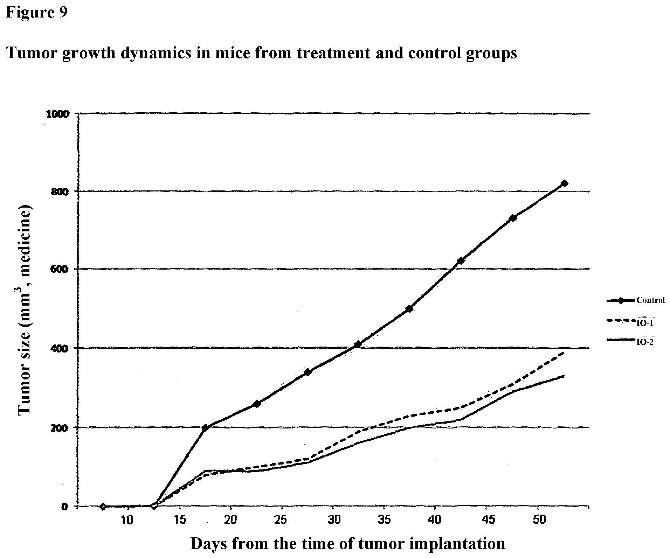

The effect of the monoclonal antibodies / saline on the growth (volume) of tumors is shown in Figure 9. The figure shows that in those mice that received monoclonal antibodies blocking FGFR1, the tumor volume was significantly smaller than in the control mice.

The tumor weight (median) in the control group was significantly higher compared to the treatment groups (p <0.001). The number of lung metastases was also significantly higher in the control mice (p <0.01).

Those mice that received neutralizing monoclonal antibodies beginning from the first week after inoculation with the Caki-1 cells, the rate of tumor growth was significantly slower than in mice injected with saline.

Based on these data, it was concluded that the method of suppressing tumor growth in vivo by blocking the FGF/FGFR1 pathway (by blocking FGFR1 domains II and IIIc) was effective.

## Claims

1. A monoclonal antibody for use in a method of treatment of renal cancer, wherein the antibody binds to fibroblast growth factor receptor 1 (FGFR1) and antagonizes the interaction of fibroblast growth factor and FGFR1, and wherein the antibody is capable of simultaneously binding to domains II and IIIc of FGFR1.

2. A monoclonal antibody for use in a method of treatment of renal cancer , wherein the antibody binds to fibroblast growth factor receptor 1 (FGFR1) and antagonizes the interaction of fibroblast growth factor and FGFR1, and wherein the antibody is capable of binding to a complex of FGFR1 and heparansulfate.

3. The antibody for use according to claim 1 or 2, **characterized in that** the antibody inhibits the mitogenic activity of *FGFR1* or its complex with heparan sulfate by not less than 90%.

4. The antibody for use according to any one of claims 1 to 3, **characterized in that** the antibody comprises an amino acid sequence of the heavy chain Fc domain of any one of: IgA, IgD, IgE, IgG1, IgG2, IgG3, IgG4 or IgM.

5. The antibody for use according to any one of claims 1 to 3, **characterized in that** the antibody comprises a human Fc domain and/or a mouse Fv domain.

6. The antibody for use according to any one of claims 1 to 3, **characterized in that** the antibody is a human antibody.

7. The antibody for use according to any one of claims 1 to 3, **characterized in that** the antibody in addition comprises a non-immunoglobulin polymer.

8. The antibody for use according to any one of claims 1 to 3, **characterized in that** the antibody in addition comprises a cytotoxic moiety or an amino acid sequence of a cytokine.

9. The antibody for use according to claim 8, **characterized in that** the cytotoxic moiety is a polypeptide toxin.

10. The antibody for use according to claim 9, **characterized in that** the cytotoxic moiety is capable of acting as an Fc-effector or capable of recruiting immunocompetent cells.

11. The antibody for use according to claim 10, **characterized in that** the cytotoxic moiety is a polypeptide capable of binding complement.

## Patentansprüche

1. Monoklonaler Antikörper zur Verwendung in einem Verfahren zur Behandlung von Nierenkrebs, wobei der Antikörper an den Fibroblastwachstumsfaktor-Rezeptor 1 (FGFR 1) bindet und die Wechselwirkung zwischen dem Fibroblastwachstumsfaktor und FGFR 1 antagonisiert und wobei der Antikörper zur gleichzeitigen Bindung an die Domänen II und IIIc von FGFR 1 befähigt ist.

2. Monoklonaler Antikörper zur Verwendung in einem Verfahren zur Behandlung von Nierenkrebs, wobei der Antikörper an den Fibroblastwachstumsfaktor-Rezeptor 1 (FGFR 1) bindet und die Wechselwirkung zwischen dem Fibroblastwachstumsfaktor und FGFR 1 antagonisiert und wobei der Antikörper zur Bindung an einen Komplex aus FGFR 1 und Heparansulfat befähigt ist.

3. Antikörper zur Verwendung nach Anspruch 1 oder 2, **dadurch gekennzeichnet, dass** der Antikörper die mitogene Aktivität von FGFR 1 oder seines Komplexes mit Heparansulfat um mindestens 90% inhibiert.

4. Antikörper zur Verwendung nach einem der Ansprüche 1-3, **dadurch gekennzeichnet, dass** der Antikörper eine Aminosäuresequenz der Schwerketten-Fc-Domäne von jeweils IgA, IgD, IgE, IgG1, IgG2, IgG3, IgG4 oder IgM umfasst.

5. Antikörper zur Verwendung nach einem der Ansprüche 1-3, **dadurch gekennzeichnet, dass** der Antikörper eine Fc-Domäne des Menschen und/oder eine Fv-Domäne der Maus umfasst.

6. Antikörper zur Verwendung nach einem der Ansprüche 1-3, **dadurch gekennzeichnet, dass** der Antikörper ein menschlicher Antikörper ist.

7. Antikörper zur Verwendung nach einem der Ansprüche 1-3, **dadurch gekennzeichnet, dass** der Antikörper zusätzlich noch ein Nicht-Immunoglobulin-Polymer umfasst.

8. Antikörper zur Verwendung nach einem der Ansprüche 1-3, **dadurch gekennzeichnet, dass** der Antikörper zusätzlich noch einen cytotoxischen Anteil oder eine Aminosäuresequenz eines Cytokins umfasst.

9. Antikörper zur Verwendung nach Anspruch 8, **dadurch gekennzeichnet, dass** der cytotoxische Anteil ein Polypeptidtoxin ist.

10. Antikörper zur Verwendung nach Anspruch 9, **dadurch gekennzeichnet, dass** der cytotoxische Anteil zur Wirkung als Fc-Effektor oder zur Rekrutierung von immunokompetenten Zellen befähigt ist.

11. Antikörper zur Verwendung nach Anspruch 10, **dadurch gekennzeichnet, dass** der cytotoxische Anteil ein zur Komplementbindung befähigtes Polypeptid ist.

## Revendications

1. Anticorps monoclonal pour utilisation dans une méthode de traitement du cancer du rein, dans laquelle l'anticorps se lie au récepteur 1 du facteur de croissance des fibroblastes (FGFR1) et antagonise l'interaction du facteur de croissance des fibroblastes et de FGFR1, et dans laquelle l'anticorps est capable de se lier simultanément aux domaines II et IIIc de FGFR1.

2. Anticorps monoclonal pour utilisation dans une méthode de traitement du cancer du rein, dans laquelle l'anticorps se lie au récepteur 1 du facteur de croissance des fibroblastes (FGFR1) et antagonise l'interaction du facteur de croissance des fibroblastes et de FGFR1, et dans laquelle l'anticorps est capable de se lier à un complexe de FGFR1 et de sulfate d'héparane.

3. Anticorps pour utilisation selon la revendication 1 ou 2, **caractérisé en ce que** l'anticorps inhibe l'activité mitogène de FGFR1 ou de son complexe avec le sulfate d'héparane de pas moins de 90 %.

4. Anticorps pour utilisation selon l'une quelconque des revendications 1 à 3, **caractérisé en ce que** l'anticorps comprend une séquence d'acides aminés du domaine Fc de chaîne lourde de l'une quelconque parmi : IgA, IgD, IgE, IgG1, IgG2, IgG3, IgG4 ou IgM.

5. Anticorps pour utilisation selon l'une quelconque des revendications 1 à 3, **caractérisé en ce que** l'anticorps comprend un domaine Fc humain et/ou un domaine Fv de souris.

6. Anticorps pour utilisation selon l'une quelconque des revendications 1 à 3, **caractérisé en ce que** l'anticorps est un anticorps humain.

7. Anticorps pour utilisation selon l'une quelconque des revendications 1 à 3, **caractérisé en ce que** l'anticorps comprend en plus un polymère non immunoglobulinique.

8. Anticorps pour utilisation selon l'une quelconque des revendications 1 à 3, **caractérisé en ce que** l'anticorps comprend en plus une fraction cytotoxique ou une séquence d'acides aminés d'une cytokine.

9. Anticorps pour utilisation selon la revendication 8, **caractérisé en ce que** la fraction cytotoxique est une toxine polypeptidique.

10. Anticorps pour utilisation selon la revendication 9, **caractérisé en ce que** la fraction cytotoxique est capable d'agir comme un effecteur Fc ou capable de recruter des cellules immunocompétentes.

11. Anticorps pour utilisation selon la revendication 10, **caractérisé en ce que** la fraction cytotoxique est un polypeptide capable de se lier au complément.
